(19) Europäisches Patentamt
European Patent Office
Office européen des brevets

(11) Publication number: 0 144 949 B1

# (12) EUROPEAN PATENT SPECIFICATION

(45) Date of publication of patent specification: 09.12.87

(51) Int. Cl.$^4$: C 07 C 51/353, C 07 C 53/08, C 07 C 53/122, C 07 C 53/124, C 07 C 63/06 // B01J23/46

(21) Application number: 84114649.1

(22) Date of filing: 03.12.84

## (54) Process for producing organic acids.

(30) Priority: 02.12.83 US 557265

(43) Date of publication of application: 19.06.85 Bulletin 85/25

(45) Publication of the grant of the patent: 09.12.87 Bulletin 87/50

(84) Designated Contracting States: DE FR GB IT NL

(56) References cited:
DE-A-2 236 439
DE-A-3 046 899
GB-A-1 286 224

(73) Proprietor: UNION CARBIDE CORPORATION
39 Old Ridgebury Road
Danbury Connecticut 06817 (US)

(72) Inventor: Wegman, Richard William
912 Glendale Avenue
South Charleston 25303 (US)
Inventor: Schreck, David James
5226 Sun Valley Drive
Cross Lanes 25313 (US)

(74) Representative: Wuesthoff, Franz, Dr.-Ing. et al
Patentanwälte Wuesthoff -v. Pechmann-Behrens-Goetz Schweigerstrasse 2
D-8000 München 90 (DE)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 144 949 B1

Courier Press, Leamington Spa, England.

**Description**

Background of the invention

The production of organic compounds using carbon monoxide or synthesis gas, which is a mixture of carbon monoxide and hydrogen, as reactant has been known for a significant period of time. It is well known that one can produce methanol directly from synthesis gas and that methanol can be further reacted by hydroformylation, homologation and carbonylation reactions to produce acetaldehyde, ethanol and acetic acid or its methyl ester, respectively. It is also known that esters, ethers, and other organic compounds can be reacted with carbon monoxide or synthesis gas to produce oxygenated organic compounds. The difficulties, however, have resided in the ability to carry out any one of these chosen reactions to produce the desired compound at acceptable efficiency, conversion rate and selectivity.

In almost all instances the reaction is generally catalyzed using a Group VIII transition metal compound as catalyst and a halogen as the promoter. It is known that many other metal compounds and promoters can be used. In addition, the prior art has disclosed the use of secondary activators or ligands in conjunction with the metal catalysts and promoters. These secondary activators can be other metallic salts or compounds, amines, phosphorus compounds, as well as a multitude of other compounds that have been disclosed in the published literature. Thus, a typical catalyst system contains the metal atom catalyst, promoter and, optionally, ligands, solvents and secondary activators. Though a significant amount of literature does exist describing the production of oxygenated organic compounds.

Several of the pertinent patents in this area are discussed below.

FR—A—2,317,269 discloses the production of aliphatic carboxylic acids by the reaction of an alcohol with carbon monoxide in the presence of a catalyst containing at least three essential components, iridum atom, copper atom and halogen.

EP—A1—00 18 927: there is described a process for the production of monocarboxylic acids by the carbonylation of an alcohol using a nickel catalyst, a halide and a solvent; in this reference synthesis gas is used.

EP—A1—00 45 637: there is disclosed the direct conversion of formic acid esters to their corresponding carboxylic acids without the presence of carbon monoxide using as catalyst a soluble iridium salt and an iodine promoter.

Another known procedure for producing acetic acid is the catalytic isomerization of methyl formate as shown by the reaction:

$$CH_3OOCH_3 \rightarrow CH_3COOH.$$

GB—A—12 86 224 discloses the production of acetic acid from methyl formate in the presence of carbon monoxide and a catalytic system comprising a rhodium-catalyst and a halogen promoter, i.e. methyliodide.

U.S.—A—1,697,109 discloses a vapor phase isomerization reaction carried out at 200°C to 450°C at a pressure up to 200 atmospheres using a metal oxide or acetate catalyst.

U.S.—A—2,508,513 claims an iron metal atom based catalyst, e.g. nickel, promoted with methyl iodide for the isomerization of methyl formate to acetic acid, carried out at 300°C to 400°C and a pressure up to 400 atmospheres. Carbon monoxide may be present.

U.S.—A—3,060,233, discloses the carbonylation of methanol to acetic acid using a metal of the iron group of the Periodic Table and a halide.

U.S.—A—3,769,329 discloses the production of carboxylic acids from alcohols, or the ester, ether and halide derivatives thereof, and carbon monoxide using a rhodium catalyst and a halogen component.

U.S.—A—3,798,267 relates to the conversion of methyl formate to acetic acid in the presence of a catalyst system consisting essentially of activated carbon and a halogen promoter.

U.S.—A—4,194,056 discloses the production of carboxylic acid from methyl formate using a soluble rhodium catalyst, halogen promoter and carbon monoxide.

U.S.—A—4,212,989, describes a process for producing carboxylic acids or their esters by reacting an alcohol or an ether with carbon monoxide using a Group VIII metal catalyst and an iodine promoter.

GB—A—1,293,193, relates to the direct conversion of formic acid esters to the corresponding carboxylic acids, in the presence of carbon monoxide, a catalyst that is a Group IIb or VIII metal and an organic polar solvent.

Japanese Patent Publication 50-16773, discloses the production of an organic acid from the corresponding formic acid ester in the presence of carbon monoxide using a catalyst system containing cobalt, iron or mercury and a halogen plus an alkali metal salt of a lower aliphatic carboxylic acid, triamine or cyclic amine.

Japanese Patent Publication 51-65703, discloses the reaction of methyl formate in the presence of carbon monoxide using a system containing a rhenium catalyst and halogen compound to produce acetic acid.

Japanese Patent Publication 56-22745, discloses the isomerization of a formic acid ester to the corresponding acid in the presence of carbon monoxide, palladium atom, halogen and base.

Japanese Patent Application No. 56-73040, relates to a process for producing acetic acid by isomerizing

methyl formate in the presence of carbon monoxide using a nickel catalyst, an iodine compound and an organic nitrogen compound.

Japanese Patent Application 56-83439, discloses a method for producing acetic acid by heating methyl formate and carbon monoxide in contact with a catalyst containing palladium, ruthenium and/or iridium metal atom and a halide promoter.

It can be seen that the prior art contains many disclosures dealing with the catalytic production of acetic acid. One of the disadvantages in many of these references is the presence of water with the eventual need to remove it from the desired organic acid product. This removal is both complicated and costly. Other disadvantages often include the simultaneous occurrence of other reactions leading to the formation of by-products, such as, dimethyl acetal, methyl acetate, ethanol, etc. These reactions compete with the organic acid production resulting in low conversion rate and selectivity to organic acid.

Many processes employed for the production of organic acids use a catalyst system containing a source of metal atom and a source of halide atom. The alkali metal halides are often mentioned as suitable halide sources, but no distinction is made between any specific one of the alkali metal halides or between any other halogen compound.

## Summary of the invention

A catalyst system and process for the production of organic acids at high efficiency, selectivity and conversion rate by the reaction of mixtures of an organic ester, such as methyl acetate and formic acid has been found. The catalyst system charged to the reactor contains Groupe VIII transition metal or compounds thereof, preferably rhodium, and lithium iodide or bromide in a mole ratio of from 1:1 to 1:1000, and optionally an organic ligand. The use of lithium iodide or bromide in this system within the ranges defined results in unexpectedly high efficiency, high conversion rate or activity and high selectivity not heretofore achieved, particularly in the production of essentially anhydrous acetic acid from formic acid and methyl acetate.

## Description of the invention

In the catalytic reactions to produce oxygenated organic compounds there are several criteria required of the catalyst. The catalyst must be as stable as possible, it should have a high activity or conversion rate, and it should have as high a selectivity for the desired product as possible.

Stability of the catalyst relates to how long the catalyst remains functional before either breaking down or losing its catalytic effect.

Activity or conversion rate relates to the amounts of reactants the catalyst converts to product per unit of time, generally expressed in g · mole per liter per hour (g mole/l · h).

Selectivity relates to the quantity of desired product produced, generally expressed in mole percent, based on the total amount of both desired products and undesired products produced.

The goal to be achieved is high values for all three criteria and continued efforts are being made to find new catalyst compositions to reach this goal without having a significant detrimental effect on the overall process. Toward this goal the prior art has developed catalyst systems containing a wide variety of metal atoms, promoters and activators, in many cases with diverse other components added. Though these catalyst systems are effective, improvement is always desirable.

The present invention is based on the unexpected and unpredictable discovery that the group VIII metal- or rhodium-lithium iodide or bromide system is an unexpectedly superior catalytic system for the production of organic acids from mixtures of formic acid and organic ester at unexpected high efficiency selectivity and conversion rate. It was also found that a ligand, $ER''_3$, can also be present as an optional component of the system. This unexpected improvement in efficiency, selectivity and conversion rate is achieved when the system's components are maintained within a defined range and when lithium iodide is present as the source of the halogen component in the system. Optionally methyl iodide and/or a solvent and/or diluent can also be present. The improved catalyst system of this invention can be portrayed as containing the components Rh-LiI-$ER''_3$, wherein Rh is the rhodium containing compound and $ER''_3$ is optionally present.

In the process of our invention mixtures of esters and formic acid are reacted using a particular catalyst system containing a group VIII metal or a compound thereof, preferably rhodium, and lithium iodide. This system produces commercially desirable organic acids at unexpectedly high efficiency, conversion rate and selectivity, with a minimum of by-products and without formation of water. The overall reaction that occurs with a simple ester is theoretically:

$$HCOOH + RCOOR' \rightarrow RCOOH + R'COOH$$

In the above formula R can be an alkyl group having from 1 to 30 carbon atoms, preferably from 1 to 15 carbon atoms and most preferably from 1 to 5 carbon atoms; an alkenyl group having from 2 to 30 carbon atoms, preferably from 2 to 15 carbon atoms and most preferably from 2 to 5 carbon atoms; or an aryl, aralkyl or alkaryl group having 6 or 10 ring carbon atoms, e.g., phenyl and naphthyl, with from 1 to 10 carbon atoms, preferably 1 to 4 carbon atoms, in the alk-moiety thereof and R' can be an alkyl group having from 1 to 3 carbon atoms, preferably 1 carbon atom. The R groups can be linear or branched and they can

be unsubstituted or substituted with groups which will not have an adverse effect on the reaction; further; the alkenyl groups can contain more than one unsaturated bond.

Illustrative of suitable esters one can mention methyl formate, ethyl formate, methyl acetate, ethyl acetate, the propyl acetates, methyl propionate, ethyl propionate, iso-propyl propionate, methyl butyrate, ethyl butyrate, iso-propyl butyrate, methyl benzoate, propyl benzoate, methyl salicylate, iso-propyl salicylate, dimethyl malonate, diethyl malonate, dimethyl succinate, diisopropyl succinate, dimethyl maleate, dimethyl phthalate, methyl cinnamate.

When R and R′ are the same, only one carboxylic acid is produced. Thus, methyl acetate produces acetic acid. When R and R′ are not the same, a mixture of organic acids is obtained. Thus, ethyl acetate or methyl propionate, or mixtures thereof, produce acetic acid and propionic acid mixtures.

The theoretical stoichiometry of the reaction is the reaction of 1 mole of formic acid per mole of ester. However, this can be varied over a molar ratio of formic acid to organic ester of from 0.1:1 or lower to 10:1 or higher.

The rhodium component of the catalyst system can be supplied from any number of sources, many of these are known to those of ordinary skill in the art. Thus, it is not necessary for an understanding thereof to specifically enumerate every suitable type and every specific compound since any of the known rhodium compounds can be used.

The essential rhodium component of the catalyst system of the present invention may be provided by introducing into the reaction zone a compound of rhodium or may be provided by introducing into the reaction zone rhodium. Among the materials which may be charged to the reaction zone to provide the rhodium component of the catalyst system of the present invention are rhodium metal, rhodium salts and oxides, organo rhodium compounds and coordination compounds of rhodium.

Specific examples of materials capable of providing the rhodium constituent of the catalyst system of the present invention may be taken from the following non-limiting partial list of suitable materials.

$RhCl_2$
$RhBr_3$
$RhI_2$
$RhCl_3 3H_2O$
$RhBr_3 3H_2O$
$Rh_2(CO)_4Cl_2$
$Rh_2(CO)_4Br_2$
$Rh_2(CO)_4I_2$
$Rh_2(CO)_8$
$Rh[(C_6H_5)_3P]_2(CO)I$
$Rh[(C_6H_5)_3P]_2(CO)Cl$
Rh metal
$Rh(NO_3)_3$
$RhCl[(C_6H_5)_3P]_2(CH_3I)_2$
$Rh(SnCl_3)[(C_6H_5)_3P]_2$
$RhCl(CO)[(C_6H_5)_3As]_2$
$RhI(CO)[(C_6H_5)_3Sb]_2$
$[(n\text{-}C_4H_9)_4N][Rh(CO)_2X_2]$ where X=Cl-, Br-, I-
$[(n\text{-}C_4H_9)_4As]_2[Rh(CO)_2Y_4]$ where X=Br-, I-
$[(n\text{-}C_4H_9)_4P][Rh(CO)I_4]$
$Rh[(C_6H_5)_3P]_2(CO)Br$
$Rh[(n\text{-}C_4H_9)_3P]_2(CO)Br$
$Rh[(n\text{-}C_4H_9)_3P]_2(CO)I$
$RhBr[(C_6H_5)_3P]_3$
$RhI[(C_6H_5)_3P]_3$
$RhCl[(C_6H_5)_3P]_2$
$RhCl[(C_6H_5)_3P]_3H_2$
$[(C_6H_5)_3P]_3Rh(CO)H$
$Rh_2O_3$
$[Rh(C_3H_4)_2Cl]_2$
$K_4Rh_2Cl_2(SnCl_2)_4$
$K_4Rh_2Br_2(SnBr_3)_4$
$K_4Rh_2I_2(SnI_2)_4$

In addition, one can use the other Group VIII transition metals comprising the iron triad, i.e., iron, ruthenium, osmium; the cobalt triad, i.e., cobalt, rhodium, iridium; or the nickel triad, i.e., nickel, palladium, platinum. Though these will catalyze the reaction, the preferred metals are nickel and rhodium, with the most preferred being rhodium.

The rhodium or Group VIII transition metal concentration can vary over a wide range. Enough metal atom must be present to achieve reasonable reaction rates; however, excess may on occasion result in

undesired by-products formation. For simplicity, the rhodium atom will be used in the specification with the understanding that it also applies to the other transition metals of Group VIII. The mole ratio of rhodium to ester can vary from 1:25 to 1:40,000, the preferred range is from 1:50 to 1:5,000, with the most preferred range being from 1:100 to 1:2,000. The amount used is not a critical feature in this invention.

The second component of the catalyst system is lithium iodide or bromide. It can be charged directly, or it can be formed in situ by any combination of lithium compound and iodine component that will result in the formation of lithium iodide or bromide during the reaction. Lithium bromide can also be used but the iodide is preferred. The presence of lithium iodide or lithium bromide is a critical feature of this invention. Direct charge of lithium iodide is the preferred form. However, any convenient combination of compounds for in situ formation of lithium iodide can be used. This includes the use of lithium carboxylates, carbonates and the like with a halogen compound. A suitable combination for in situ formation is lithium carboxylate and an alkyl halide. Though any combination of carboxylate and halide can be used, if the feedstock is RCOOR' then the preferred combination is RCOOLi and R'I. For example, if the ester is methyl acetate, then the preferred combination is lithium acetate and methyl iodide.

Sufficient lithium iodide or bromide must be present to exert a promoting effect on the reaction and to result in high efficiency, conversion rate and selectivity to the corresponding acid. The mole ratio of Rh:LiI or LiBr can vary over a wide range. A Rh:LiI or LiBr mole ratio of from 1:1 to 1:1000 can be employed, the preferred range is from 1:2 to 1:450 and most preferably it is from 1:8 to 1:150.

As indicated, an organic ligand of the general formula $ER''_3$ can optionally be present in the reaction system. The use of such ligands is known, as are their identities, to those skilled in this art. In this formula E represents a Group VA element, e.g., N, P, As, Sb and Bi, and R'' represents an organic moiety. The ligand can serve as a catalyst stabilizer and/or to further enhance efficiency, conversion rate and selectivity, especially when the reaction is carried out at higher temperatures, for example at about 200°C or above. The ligand also serves to inhibit equipment corrosion in some instance. However, the use of a ligand is not mandatory and the reaction can be carried out without it.

A large number of organic ligands is known and any of these can be used provided they do not have an adverse effect on the reaction. Among those of particular utility are the tertiary amines and the tri- and pentavalent phosphorus compounds. Though those skilled in the art know these compounds, illustrative of suitable compounds one can mention triethylphosphine, tributylphosphine, tri-2-ethylhexylphosphine, triphenylphosphine, tri(4-methoxyphenyl)phosphine, tri-p-tolylphosphine, tri(3-chlorophenyl)phosphine, diphenyl hexylphosphine, dimethyl (3 - methoxyphenyl)phosphine, dibutyl stearylphosphine, tribenzylphosphine, dipropyl phenylphosphine, ethyl dipropylphosphine, tricyclohexylphosphine, cyclohexyl dibutylphosphine, propyl diphenylphosphine, dipropyl phenylphosphine, phenyl diethylphosphine, tridecylphosphine, trioctadecylphosphine, tribenzylphosphine, methyl diethylphosphine, ethyl diphenylphosphine, tolyl diethylphosphine, cyclohexyl diethylphosphine, diethyl cyclohexylphosphine, bis - (diphenylphosphino)ethane, bis - (diethylphosphino)propane, bis - (diphenylphosphino)butane, bis - (diethylphosphino)octane, trimethylamine, triethylamine, tri - n - butylamine, tri-t-butylamine, tri - 2 - ethylhexylamine, methyl dibutylamine, tridodecylamine, tristearylamine, ethyl dibutylamine, tricyclohexylamine, triphenylamine, tri(4-methoxyphenyl)amine, tri(p-chlorophenyl)amine, dibutyl phenylamine, dipentyl cyclopentylamine, ethyl diphenylamine, trinaphthylamine, tri-p-tolylamine, tri-benzylamine, tri(3-methylcyclohexyl)amine, and the arsines, stibines and bismuthines corresponding to the above-identified phosphines and amines. They can be used singly or, if one desired, mixtures containing 2 or more ligands can be used. One can also employ a phosphine oxide or phosphite corresponding to the above phosphines as the ligand; these are also well known.

The concentration of ligand charged can vary from a molar ratio of ligand to rhodium of from 50:1 to 1:50, preferably from 10:1 to 1:10, most preferably 3:1 to 1:1.

In addition to the ligand one can optionally have a solvent present. Many essentially inert solvents are known as useful, essentially inert diluents and illustrative thereof one can mention 1,4-dioxane, the polyethylene glycol di-ethers or esters, diphenyl ether, sulfolane, toluene, carboxylic acids as well as any other diluent or solvent which does not interfere with the reaction to any significant extent. The reaction is preferably carried out in the absence of any solvent or diluent other than those required to introduce reactants or catalyst components.

The reaction is carried out at a temperature of from 50°C to 350°C, preferably from 120°C to 220°C and most preferably from 140°C to 200°C. When the reaction is carried out at temperatures above 200°C in the presence of an $ER''_3$ ligand, the preferred ligands are the phosphines, especially triphenyl phosphine.

The pressure of the reaction can be from 3,45 to 690 bar (50 psig to 10,000 psig), preferably from 6,9 to 414 bar (100 psig to 6,000 psig), most preferably from 13,8 to 34,5 bar (200 psig to 500 psig).

The reaction time varies depending upon the reaction parameters, reactor size and charge, and the individual components employed at the specific process conditions. The reaction can be a batch or continuous reaction.

The experiments and examples detailed below were carried out in a Hasteloy® steel autoclave reactor having a volume of 300 ml, which was equipped with temperature and pressure sensing means, heating and cooling means, agitator and inlet and outlet means for introducing and removing components from the reactor. The autoclaves used in synthesis gas reactions are well known in the art and can be used in this process.

Prior to charging the reactants the autoclave was washed with methanol at 100°C under a nitrogen gas pressure of 34,5 to 69 bar (500 to 1,000 psig) by agitating for 30 minutes. The autoclave was drained, rinsed with dry acetone, and dried with nitrogen or any other inert gas. The liquid components were charged to the cleaned autoclave first and then the solid components were added and stirred. The autoclave was closed and purged with nitrogen. The autoclave contents were heated to the selected temperature, with agitation (usually 750 rpm), in about 45 minutes. The reaction was maintained at the prescribed temperature for a specified period of time, usually between 0.5 to 5 hours.

At the end of the reactor run, the contents were cooled, generally to about 10°C. A vapor phase sample was taken for gas chromatography analysis; the gas phase was vented through two dry-ice acetone traps and then through a 10 liter saturated solution of calcium hypochlorite to remove metal carbonyls, if formed. The reactor was pressurized three times with nitrogen, 6,2 bar (90 psig) and vented through the same system.

The residual reactor contents were dumped into a chilled pressure bottle and sealed. Subsequent analysis was performed using a Hewlett-Packard Model 5880 gas chromatograph equipped with a 3,2 mm (one-eighth inch) diameter by (ten feet) 3 m long column packed with Chromosorb 101.

The following examples serve to further illustrate this invention. In the examples the term "AcAc" means "acetylacetonate".

Example 1

The autoclave was charged with 2.06 g of $Rh(CO)_2AcAc$ (8 mmoles), 82 g of formic acid (1.8 moles), 17.1 g of lithium iodide (128 mmoles) and 70 g of methyl acetate (0.95 mole). Following the procedure described above the reaction was carried out at 180°C for 3 hours, under nitrogen, at ambient autoclave pressure. Analysis indicated the following products present in the reaction product:

| | |
|---|---|
| Acetic acid | 1.63 moles |
| Formic acid | 0.1 mole |
| Methyl acetate | Trace |

The acetic acid yield was 86% of theoretical and its conversion rate to acetic acid was 3.6 g moles/l · h. There was no indication of water formation.

In the following examples the same procedure was employed using different organic esters as feedstock to produce the acids indicated at high yield and rate; water was not formed.

| Example | Feedstock | Product(s) |
|---|---|---|
| 1a | Propyl propionate | Propionic acid<br>Butanoic acid |
| 1b | Methyl propionate | Propionic acid<br>Acetic acid |
| 1c | Ethyl benzoate | Benzoic acid<br>Propionic acid |
| 1d | Methyl formate | Formic acid<br>Acetic acid |
| 1e | Ethyl butyrate | Butanoic acid<br>Propionic acid |

**Claims**

1. A process for the production of organic carboxylic acids of the formulas RCOOH and R'COOH by a catalytic reaction of an organic ester of the formula RCOOR' wherein R is an alkyl group having from 1 to 30 carbon atoms, or aryl, aralkyl or alkaryl group having 6 to 10 ring carbon atoms with from 1 to 10 carbon atoms in the alk- moiety thereof, or an alkenyl having from 2 to 30 carbon atoms, and R' is an alkyl group having 1 to 3 carbon atoms, in contact with a homogeneous catalytic system comprising a group VIII transition metal-catalyst and a halogen promoter characterized in that a mixture of the organic ester and formic acid is reacted in contact with the catalytic system, wherein the halogen promoter is lithium-iodide or -bromide and the mole ratio of the metal catalyst to the halogen promoter is from 1:1 to 1:1000.

2. The process as claimed in claim 1 wherein the group VIII transition metal is rhodium.

3. The process as claimed in claim 1 or 2 wherein the temperature is from 50°C to 350°C and the pressure is from 3,45 to 690 bar.

4. The process as claimed in claims 1 to 3 wherein the mole ratio of Rh:LiI is from 1:8 to 1:150.

5. The process as claimed in claims 1 to 4 wherein the R groups of the organic ester are alkyl groups

having from 1 to 15 carbon atoms, alkenyl groups having from 2 to 15 carbon atoms, or aryl, aralkyl or alkaryl groups having from 6 to 10 ring carbon atoms and from 1 to 4 carbon atoms in the alk-moiety thereof.

6. The process as claimed in claim 1 to 5 wherein the R' group is methyl.

7. The process as claimed in claims 1 to 6 wherein the organic ester is methyl acetate.

8. The process as claimed in claims 1 to 7 wherein an organic ligand of formula $ER_3''$ is present, wherein E is nitrogen, phosphorus, arsenic, antimony and bismuth and R'' is an organic moiety.

9. The process as claimed in claim 8 wherein the ligand is a tertiary phosphine.

**Patentansprüche**

1. Verfahren zur Herstellung organischer Carbonsäuren der Formeln RCOOH und R'COOH durch katalytische Umsetzung eines organischen Esters der Formel RCOOR', worin R eine Alkylgruppe mit 1 bis 30 Kohlenstoffatomen oder eine Aryl-, Aralkyl- oder Alkarylgruppe mit 6 bis 10 Ringkohlenstoffatomen und 1 bis 10 Kohlenstoffatomen im Alk-Teil oder eine Alkenylgruppe mit 2 bis 30 Kohlenstoffatomen ist und R' eine Alkylgruppe mit 1 bis 3 Kohlenstoffatomen sein kann, mit Hilfe eines homogenen katalytischen Systems, welches ein Übergangsmetall der VIII. Gruppe als Katalysator und einen Halogen-Promotor enthält, dadurch gekennzeichnet, daß ein Gemisch des organischen Esters und Ameisensäure mit einem katalytischen System umgesetzt wird, in welchem der Halogen-Promotor Lithium-iodid oder -bromid ist und das Molverhältnis Metall-Katalysator zu Halogen-Promotor zwischen 1:1 und 1:1000 liegt.

2. Verfahren nach Anspruch 1, worin das Übergangsmetall der VIII. Gruppe Rhodium ist.

3. Verfahren nach Anspruch 1 oder 2, das bei 50 bis 350°C unter einem Druck von 3,45 bis 690 bar durchgeführt wird.

4. Verfahren nach Anspruch 1 bis 3, worin das Molverhältnis Rh:LiJ 1:8 bis 1:150 beträgt.

5. Verfahren nach Anspruch 1 bis 4, worin R der organischen Ester/Alkylgruppen mit 1 bis 15 Kohlenstoffatomen, Alkenylgruppen mit 2 bis 50 Kohlenstoffatomen oder Aryl-, Aralkyl- oder Alkarylgruppen mit 6 bis 10 Ringkohlenstoffatomen und 1 bis 4 Kohlenstoffatomen in dem Alk-Teil sind.

6. Verfahren nach Anspruch 1 bis 5, worin R' Methyl ist.

7. Verfahren nach Anspruch 1 bis 6, worin der organische Ester Methylacetat ist.

8. Verfahren nach Anspruch 1 bis 7, worin ein organischer Ligand der Formel $ER_3''$ vorhanden ist, wobei E Stickstoff, Phosphor, Arsen, Antimon oder Bismut und R'' eine organische Gruppe sind.

9. Verfahren nach Anspruch 8, worin der Ligand ein tertiäres Phosphin ist.

**Revendications**

1. Procédé de production d'acides organiques carboxyliques de formules RCOOH et R'COOH par une réaction catalytique d'un ester organique de formule RCOOR', dans laquelle R est un groupe alkyle ayant 1 à 30 atomes de carbone ou un groupe aryle, aralkyle ou alkaryle ayant 6 à 10 atomes de carbone dans le noyau avec 1 à 10 atomes de carbone dans leurs portions alkyle, ou un groupe alcényle ayant 2 à 30 atomes de carbone, et R' est un groupe alkyle ayant 1 à 3 atomes de carbone, au contact d'un système catalytique homogène comprenant un catalyseur formé d'un métal de transition du groupe VIII et un groupe promoteur halogéné, caractérisé en ce qu'on fait réagir un mélange de l'ester organique et d'acide formique au contact du système catalytique, le promoteur halogéné est l'iodure ou le bromure de lithium et le rapport molaire du catalyseur métallique au promoteur halogéné va de 1:1 à 1:1000.

2. Procédé suivant la revendication 1, dans lequel le métal de transition du groupe VIII est le rhodium.

3. Procédé suivant la revendication 1 ou 2, dans lequel la température va de 50 à 350°C et la pression va de 3,45 à 690 bars.

4. Procédé suivant les revendications 1 à 3, dans lequel le rapport molaire Rh:LiI va de 1:8 à 1:150.

5. Procédé suivant les revendications 1 à 4, dans lequel les groupes R de l'ester organique sont des groupes alkyle ayant 1 à 15 atomes de carbone, des groupes alcényle ayant 2 à 15 atomes de carbone ou des groupes aryle, aralkyle ou alkaryle ayant 6 à 10 atomes de carbone dans le noyau et 1 à 4 atomes de carbone dans leurs portions alkyle.

6. Procédé suivant les revendications 1 à 5, dans lequel le groupe R' est le groupe méthyle.

7. Procédé suivant les revendications 1 à 6, dans lequel l'ester organique est l'acétate de méthyle.

8. Procédé suivant les revendications 1 à 7, dans lequel on opère en présence d'un ligand organique de formule $ER_3''$, dans laquelle E est l'azote, le phosphore, l'arsenic, l'antimoine et le bismuth et R'' est un groupement organique.

9. Procédé suivant la revendication 8, dans lequel le ligand est une phosphine tertiaire.